# FASCICULE DE BREVET EUROPEEN

(11) **EP 4 351 606 B1**
(45) Date de publication et mention de la délivrance du brevet: **05.08.2026**
(21) Numéro de dépôt: 22733357.2
(22) Date de dépôt: 08.06.2022
(51) Int. Cl.: A61K 35/747, A61K 35/744, A61K 31/702, A61P 3/04, A61P 3/10

(54) **PROBIOTIQUE COMPRENANT UNE SOUCHE L. CASEI ET SES UTILISATIONS DANS DES SITUATIONS DE DENUTRITION**
PROBIOTIKUM MIT EINEM L. CASEI-STAMM UND SEINE ANWENDUNG BEI MANGELERNÄHRUNG
PROBIOTIC CONTAINING AN L. CASEI STRAIN AND ITS USES IN MALNUTRITIONAL SITUATIONS

(30) Priorité: 09.06.2021 FR 2106084
(43) Date de publication de la demande: 17.04.2024
(73) Titulaire: Institut National de la Recherche pour l'Agriculture, l'Alimentation et l'Environnement, 75007 Paris (FR); Institut National des Sciences et Industries du Vivant et de l'Environnement, 91120 Palaiseau (FR); Université Clermont Auvergne, 63001 Clermont-Ferrand (FR); Institut d'Enseignement Supérieur et de Recherche en Alimentation, Santé Animale, Sciences Agronomiques et de l'Environnement, 69280 Marcy l'Etoile (FR); ASSISTANCE PUBLIQUE HOPITAUX DE PARIS, 75004 Paris (FR); Université de Paris Cité, 75006 Paris (FR); Université Paris-Saclay, 91190 Gif-sur-Yvette (FR)
(72) Inventeur: THOMAS, Muriel, 91430 Igny (FR); SAVARY-AUZELOUX, Isabelle, 63000 CLERMONT-FERRAND (FR); JARZAGUET, Marianne, 63410 LOUBEYRAT (FR); MAYEUR, Camille, 21200 BEAUNE (FR); BORNES, Stéphanie, 15130 TEISSIERES-LES-BOULIES (FR); GIRON, Muriel, 63450 LE CREST (FR); DARDEVET, Dominique, 63170 PERIGNAT LES SARLIEVES (FR); JOLY, Françoise, 75017 PARIS (FR); CHASSARD, Christophe, 15000 AURILLAC (FR)
(74) Mandataire: Ipsilon
(86) Numéro de dépôt international: PCT/EP2022/065569
(87) Numéro de publication internationale: WO 2022/258694

(56) Documents cités:
- US-A1- 2017 080 034
- SALLES BÁRBARA IZABEL MORAES ET AL: "Probiotics supplementation and insulin resistance: a systematic review", vol. 12, no. 1, 1 December 2020 (2020-12-01), pages 98, XP055890308, Retrieved from the Internet <URL:https://www.ncbi.nlm.nih.gov/pmc/articles/PMC7656736/pdf/13098_2020_Article_603.pdf> DOI: 10.1186/s13098-020-00603-6
- SHUKLA GEETA ET AL: "Prebiotic inulin supplementation modulates the immune response and restores gut morphology inGiardia duodenalis-infected malnourished mice", PARASITOLOGY RESEARCH, SPRINGER BERLIN HEIDELBERG, BERLIN/HEIDELBERG, vol. 115, no. 11, 30 July 2016 (2016-07-30), pages 4189 - 4198, XP036072911, ISSN: 0932-0113, [retrieved on 20160730], DOI: 10.1007/S00436-016-5196-X
- KERAC M ET AL: "Probiotics and prebiotics for severe acute malnutrition (PRONUT study): a double-blind efficacy randomised controlled trial in Malawi", THE LANCET, ELSEVIER, AMSTERDAM, NL, vol. 374, no. 9684, 11 July 2009 (2009-07-11), pages 136 - 144, XP026284908, ISSN: 0140-6736, [retrieved on 20090709], DOI: 10.1016/S0140-6736(09)60884-9
- MURIEL GIRON ET AL: "Skip to Main content Skip to Navigation How to reduce Sarcopenia in the Undernourished Elderly: Enrichment of Food with "Energy Saver" Probiotics", 1 December 2021 (2021-12-01), XP055890474, Retrieved from the Internet <URL:https://hal.laas.fr/UNH/hal-03456032> [retrieved on 20220211]

## Description

### Domaine technique

La présente description se rapporte au domaine du traitement de situations de dénutrition, pathologiques ou non pathologiques, entraînant une perte de masse musculaire et/ou impliquant la présence d'une dérégulation de l'insulino-sensibilité.

### Technique antérieure

La dénutrition affecte un nombre croissant d'humains, y compris dans les pays développés. Selon un rapport de la Société Francophone, Nutrition Clinique et Métabolisme, une prévalence de la dénutrition allant de 5% à 10% de la population peut être estimée. Elle est supérieure dans la population française âgée, dont la proportion de personnes âgées de plus de 75 ans atteindra une personne sur cinq en 2050 (Données de l'INSEE), dont deux millions d'individus déments (Projection de la cohorte Paquid, 2003). Il existe ainsi une population âgée dénutrie et fortement sarcopénique, généralement dépendante, vivant en institution, et en état de sous nutrition chronique (Buckinx et al., Archives of Public Health - Archives belges de santé publique, Vol. 73(1) : 19).

Comme cela est connu, la dénutrition résulte d'un déséquilibre entre (i) les besoins nutritionnels d'un sujet et (ii) les apports nutritionnels dudit sujet. Les besoins nutritionnels d'un sujet sain, à un moment donné, peuvent varier par exemple selon l'intensité de l'exercice physique pratiqué quotidiennement, ou encore par la nécessité d'assurer une croissance physique appropriée. Les besoins nutritionnels d'un sujet malade, à un moment donné, peuvent également par exemple être augmentés du fait de la nécessité de contrecarrer une pathologie, ce qui peut conduire à terme à un état de cachexie, le cas échéant chez un sujet affecté d'un cancer. Ainsi, qu'il s'agisse d'un sujet sain ou d'un sujet affecté d'une pathologie, il est remédié à un état de dénutrition par l'apport audit sujet de nutriments dont la quantité et/ou la qualité sont adaptées à l'objectif nutritionnel ou à visée médicale qui est poursuivie.

Ainsi, la dénutrition est définie dans l'état de la technique comme étant un « état d'un organisme en déséquilibre nutritionnel » (Voir, « Diagnostic de la dénutrition de l'enfant et de l'adulte - Méthode Recommandations pour la pratique clinique », Novembre 2019, Haute Autorité de Santé et Fédération Française de Nutrition), ledit déséquilibre étant caractérisé par un bilan énergétique et/ou protéique négatif. Cette définition englobe une pluralité de situations pouvant conduire à un état de dénutrition, comme un déficit d'apport isolé en nutriments, un accroissement des dépenses ou des pertes induisant un déséquilibre. Le déséquilibre inhérent à la dénutrition conduit à des effets délétères sur le corps, accompagnés de changements mesurables des fonctions corporelles et/ou de la composition corporelle, ces changements pouvant être associés à une aggravation de l'évolution de maladies desquelles peut être affecté le sujet.

Il existe deux grandes formes de dénutrition, chacune à l'extrémité d'un même continuum physiopathologique. Les deux formes peuvent co-exister. Des critères cliniques ont été précisés pour ces deux formes. À l'une des extrémités, le marasme, sans œdème, par carence d'apport isolée, dont la traduction est principalement anthropométrique (amaigrissement et/ou IMC (Indice de Masse Corporelle) faible), par exemple lors de l'anorexie mentale non décompensée. À l'autre extrémité, la forme hyper-catabolique avec œdèmes. Cette forme associe une carence d'apport et un stress métabolique, à l'origine d'une perte protéique, d'une perte de masse et de fonction musculaires. La traduction du stress métabolique est principalement biologique (syndrome inflammatoire et/ou albumine diminuée).

Les conséquences de la dénutrition sont multiples et représentent un enjeu de santé publique : allongement des durées d'hospitalisation, augmentation des risques d'infection nosocomiale, de chute, de fracture ou d'entrée dans la dépendance, déficit immunitaire, dépression, aggravation des maladies chroniques avec pour conséquence ultime une augmentation de la mortalité. Les situations étiologiques favorisant la dénutrition sont les situations où il existe une réduction de la prise alimentaire, un trouble de l'absorption intestinale et/ou une situation d'hypercatabolisme protéique avec ou sans syndrome inflammatoire que l'on nomme situation d'agression. Le diagnostic de dénutrition repose donc sur la présence d'un critère étiologique et de la présence d'au moins un critère phénotypique. Ainsi, les nouveaux critères HAS 2019 ont émis les critères suivants pour les sujets entre 18 et 70 ans :
- Perte de poids ≥ 5 % en 1 mois ou ≥ 10 % en 6 mois ou ≥ 10 % par rapport au poids habituel avant le début de la maladie
- les sujets ayant un indice de masse corporelle (IMC) inférieur à 18.5 kg/m2
- Réduction quantifiée de la masse et/ou de la fonction musculaire.

Pour les sujets âgés de plus de 70 ans, les critères diagnostiques sont en cours de réévaluation par la HAS car maintenant considérés comme trop anciens. Mais, ils tiendront compte également de l'IMC (Indice de Masse Corporelle), de la cinétique de perte de poids et de la fonction/force musculaire. Il est important de noter que le sujet âgé est à très haut risque de présenter une dénutrition du fait de la fréquence plus élevée de comorbidités et donc d'état hypercatabolique, de troubles bucco-dentaires, de polymédication pouvant altérer la prise alimentaire. La diminution des apports alimentaires peut également être favorisée par un contexte social inadéquat. L'isolement social et/ou familial, le manque d'aide pour l'organisation des courses et la préparation des repas, l'insuffisance des ressources financières ont un impact sur la qualité des repas. La diminution des capacités psychiques (dépression, démence...) et physiques de la personne âgée entraînent une perte d'autonomie pour les actes de la vie quotidienne et peuvent participer à l'apparition d'une dénutrition qui elle-même va aggraver l'état physiologique du patient.

Aujourd'hui, l'évaluation de la masse musculaire et/ou de la force/fonction musculaire sont devenus des critères diagnostiques car de nombreux travaux montrent l'importance des conséquences de la fonte musculaire ou sarcopénie dans le pronostic des patients. La sarcopénie (mot issu du grec qui peut être traduit par « manque de chair »), initialement définie par une perte de masse squelettique musculaire, est actuellement caractérisée par une perte de masse musculaire associée à une dégradation fonctionnelle. Elle constitue un facteur de gravité de la dénutrition.

La plus grande partie des personnes dénutries sont retrouvées dans les établissements de santé. Ainsi, en France, dans un établissement de court séjour, la proportion de personnes dénutries est respectivement (i) de 20% pour les enfants, (ii) de 45% pour les adultes et (iii) de 60% pour les personnes âgées.

Toutefois, la dénutrition concerne aussi des populations d'humains qui ne sont pas directement affectées par une malnutrition provoquée par l'impossibilité d'un accès à une quantité suffisante de nourriture. Il s'agit, comme rappelé ci-dessus, notamment des personnes âgées mais aussi des personnes affectées par une pathologie entraînant une dénutrition, tels que les patients cancéreux en cours de traitement thérapeutique.

La dénutrition affecte notamment les patients cancéreux affectés d'une cachexie, laquelle est accompagnée d'une réduction de la fonction d'organe, d'une altération de l'état immunitaire et d'une diminution de la force musculaire.

Il existe aussi des situations de dénutrition chez les personnes obèses qui pratiquent un régime pouvant entraîner une carence alimentaire. Chez les personnes obèses, une situation de stress, par exemple consécutive à une intervention chirurgicale, notamment de chirurgie de l'obésité, peut entraîner une dénutrition alors que l'indice de masse corporelle est supérieur à 30. Une partie des personnes en surpoids ou obèses est affectée d'une susceptibilité accrue à développer un diabète, par exemple un diabète impliquant une insulino-résistance, ou des pathologies cardiovasculaires, et présentent une perte accrue de masse musculaire et le cas échéant une altération de leur composition corporelle, ce qui complique la détermination de traitements adaptés de l'obésité par la mise en œuvre de régimes hypocaloriques (Barazzoni et al. (2018, Obesity Facts, Vol. 11(4) : 294-305). Chez les obèses astreints à un régime alimentaire hypocalorique, il est connu que la perte de masse grasse peut être accompagnée d'une perte de masse musculaire. Salles et al. (2020, Diabetol Metab Syndr, Vol. 12 : 98) rapportent l'utilisation de certaines souches spécifiques de *L. casei.* La souche *L. casei* CCFM419 est décrite comme induisant une réduction de la valeur de HOMA-IR.

Une situation de dénutrition peut aussi survenir en cas de pathologie digestive chronique (comme les maladies inflammatoires chroniques de l'intestin ou le syndrome de grêle court), d'insuffisance rénale ou encore de bronchopathie chronique ou d'infections respiratoires. Une dénutrition peut aussi être consécutive à un mauvais état bucco-dentaire, un trouble de la déglutition, ou encore à un régime alimentaire drastique.

Une dénutrition peut également survenir chez la femme enceinte.

Les sujets sportifs ont des besoins protéiques et énergétiques considérables aux fins de soutenir une activité physique intense. Ce besoin énergétique accru, s'il n'est pas accompagné d'un régime alimentaire approprié, peut conduire à une perte de la masse musculaire. Cette diminution de la masse musculaire, consécutive à un effort physique intense, peut être compensée par l'ingestion de compléments alimentaires, notamment par la prise orale de compléments protéiques, d'apports d'acides aminés spécifiques comme la leucine, etc, l'ingestion de ces compléments alimentaires visant à préserver la masse maigre. Les sujets sportifs peuvent anticiper ces besoins protéiques et énergétiques par l'ingestion de compléments pendant et/ou après l'effort, dans l'objectif de maintenir et/ou reconstituer les réserves nutritionnelles.

Ainsi, qu'il s'agisse des personnes âgées fragiles, de patients atteints de cancers ou des individus obèses soumis à un régime hypocalorique, la problématique de la fonte musculaire est un élément clé, dans un contexte d'apport alimentaire inférieur aux besoins, tant au niveau énergétique que protéique, lequel est accompagné d'une perte de la masse grasse mais aussi d'une perte de la masse musculaire.

La prise en charge thérapeutique de personnes affectées d'une dénutrition repose sur une pluralité de principes, parmi lesquels (i) une évaluation de l'état nutritionnel, le plus rapidement possible et (ii) le choix d'une approche nutritionnelle selon les apports alimentaires spontanés de la personne dénutrie et selon la sévérité de la dénutrition. La prise en charge thérapeutique de personnes dénutries peut inclure la prise orale de compléments nutritionnels, destinés notamment à réduire la perte concomitante de masse musculaire.

Parmi les compléments nutritionnels qui ont été étudiés dans l'état de la technique, on peut citer les compléments nutritionnels comprenant des probiotiques. Plusieurs essais ont été réalisés chez des enfants dénutris. On peut, par exemple se référer à un essai clinique randomisé (étude PRONUT) visant à évaluer l'efficacité clinique et nutritionnelle d'un mélange alimentaire comprenant quatre bactéries probiotiques *(Pediococcus pentosaceus, Leuconostoc mesenteroides, Lactobacillus paracasei, Lactobacillus plantarum)* en combinaison avec quatre types de prébiotiques (inuline, pectine, son d'avoine, amido-résistant) chez des enfants malawites âgés de 5 à 168 mois (Kerac et al., 2009, Lancet, Vol. 374 : 136-144). Aucune amélioration significative de l'état nutritionnel, la mortalité, la prise pondérale, temps de guérison ou la prévalence de symptômes cliniques tels que diarrhées, fièvre ou problèmes respiratoires n'a été observée. A l'inverse, un autre essai clinique, non randomisé, a démontré qu'une supplémentation en probiotiques permettait d'augmenter le poids et la taille d'enfants indiens avec un retard de croissance (Saran et al., 2002, Nutrition, Vol. 18 : 393-396). Il peut également être fait référence aux travaux de Matsuo et al. (2013, Internal Sociéty of Exercise and Immunology, 11th symposium, Newcastle, Australia) qui ont montré, dans un modèle murin de muscle squelettique endommagé par l'injection d'une cardiotoxine, que l'administration orale de *L. casei* a provoqué une récupération de la masse musculaire et une régénération du tissu musculaire. Ni et al. (2019, Mol Nutr Food Res, Vol. 63 : 1900603) ont montré qu'une supplémentation de souris jeunes ou âgées avec *L. casei* ou *Bifidobacterium longum* permettait d'accroître la fonction et la force musculaire. On peut aussi citer la demande de brevet US 2017/080034, qui a trait à l'utilisation de compositions comprenant des *Lactobacillus* pour stimuler la croissance chez des sujets malnutris.

Concernant la désignation de certains probiotiques, et en particulier des bactéries du genre *Lactobacillus,* il est important de noter les changements récents intervenus dans leur classification taxonomique, comme cela est rapporté dans l'article de Zheng et al. (2020, Int. J Syst Evol Microbiol, Vol. 70 : 2782-2858). Ainsi, certains probiotiques du genre *Lactobacillus* documentés dans la littérature publiée précédemment à ces changements taxonomiques sont aujourd'hui désignés selon la nouvelle nomenclature en vigueur.

A titre illustratif, certains changements de désignation d'espèces de *Lactobacillus* apportés selon la nouvelle classification en vigueur sont décrits dans le Tableau 1 ci-dessous.

**[Tableau 1]**

| **Ancienne désignation** | **Nouvelle désignation** |
|---|---|
| *Lactobacillus acidophilus* | *Lactobacillus acidophilus* |
| *Lactobacillus gasseri* | *Lactobacillus gasseri* |
| *Lactobacillus helveticus* | *Lactobacillus helveticus* |
| *Bulgaricus lactobacille* | *Lactobacillus delbrueckii subsp. bulgaricus* |
| *Lactobacillus brevis* | *Levilactobacillus brevis* |
| *Lactobacillus casei* | *Lacticaseibacillus casei* |
| *Lactobacillus fermentum* | *Limosilactobacillus fermentum* |
| *Lactobacillus paracasei* | *Lacticaseibacillus paracasei* |
| *Lactobacillus plantarum* | *Lactiplantibacillus plantarum* |
| *Lactobacillus reuteri* | *Limosilactobacillus reuteri* |
| *Lactobacillus rhamnosus* | *Lacticaseibacillus rhamnosus* |
| *Lactobacillus salivarius* | *Ligilactobacillus sativairius* |

### Tableau 1: Correspondance entre l'ancienne et la nouvelle désignation taxonomique de certaines espèces de Lactobacillus.

La correspondance entre des désignations taxonomiques selon l'ancienne et la nouvelle nomenclature peut aussi être retrouvée sur internet à l'adresse suivante : http://lactobacillus.ualberta.ca/.

Concernant le traitement de la dénutrition concomitante à un cancer, on peut citer l'étude de Bindels et al. (2012, PloS one, Vol. 7(6) : e37971) qui ont étudié le microbiote intestinal dans un modèle de souris cachectiques affectées d'une leucémie aigüe. Le traitement de ces souris avec une supplémentation orale contenant *Lactobacillus reuteri* a entraîné une diminution de marqueurs de l'atrophie musculaire dans les muscles gastrocnémiens et tibiaux antérieurs. Par ailleurs, Varian et al. (2016, Oncotarget, Vol. 7(11): 11803-11816) ont montré, dans un modèle murin de cachexie, qu'une supplémentation avec une bactérie commensale, *L. reuteri,* permettait de réduire les indices systémiques de l'inflammation et d'inhiber la cachexie.

Également, la supplémentation avec certains probiotiques, telles que certaines souches de *Lactobacillus,* de *Bifidobacterium,* de *Clostridium* ou encore de *Akkermansia, Blautia, F. prausnitzii* peut apporter des changements bénéfiques dans des situations d'insulino-résistance (Izabel et al., 2020, Diabetol Metab Syndr, Vol. 12 : 98).

Il demeure un besoin pour des traitements de sujets dénutris, y compris de sujets âgés, de sujets obèses et/ou diabétiques, de sujets dont l'exercice physique a entraîné une perte de masse musculaire ou encore de sujets cancéreux affectés d'une cachexie.

### Résumé de l'invention

La présente description concerne une composition comprenant des bactéries de la souche *L. casei* SGC-K déposée à la Collection Nationale de Cultures de Microorganismes de l'Institut Pasteur le 8 mars 2021 sous le n° CNCM I-5663.

Dans certains modes de réalisation, les bactéries de la souche *L. casei* SGC-K sont combinées avec les bactéries d'une ou plusieurs autres souches bactériennes probiotiques, ce qui inclut d'autres souches bactériennes probiotiques d'origine commensale.

Les autres souches bactériennes probiotiques peuvent être choisies parmi les genres *Bifidobacterium, Lactobacillus (et les nouvelles espèces décrites des nouveaux genres décrits), Lactococcus, Enterococcus, Streptococcus, Kluyveromyces, Saccharomyces, Faecalibacterium, Blautia, Christensenella, Bacteroides, Eubacterium, Roseburia, Coprococcus* et leurs combinaisons.

Les autres souches bactériennes peuvent être choisies parmi les espèces *Bifidobacterium longum, Bifidobacterium lactis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus salivarius, Lactococcus lactis, Enterococcus faecium*, *Enterococcus faecalis*, *Saccharomyces cerevisiae, Saccharomyces boulardii, Faecalibacterium prausnitzii* ou leurs mélanges, de préférence choisis dans le groupe constitué par *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM I- 3446), *Bifidobacterium breve* souche A, *Lactobacillus paracasei* NCC2461 (CNCM 1-2116), *Lactobacillus johnsonii NCC* 533 NCC533 (CNCM 1-1225), *Lactobacillus rhamnosus* GG (ATCC53103), *Lactobacillus rhamnosus* NCC4007 (CGMCC 1. 3724), *Enterococcus faecium* SF 68 (NCC2768 ; NCIMB10415), et leurs combinaisons.

Dans certains modes de réalisation, la composition comprend de plus un ou plusieurs prébiotiques.

Dans certains modes de réalisation, les probiotiques sont choisis parmi les oligosaccharides contenant du fructose, du galactose, du mannose ; les fibres alimentaires, en particulier les fibres fermentescibles, les fibres de soja ; l'inuline ; et leurs combinaisons.

Dans certains modes de réalisation, les prébiotiques sont choisis parmi les fructooligosaccharides (FOS), les galacto-oligosaccharides (GOS), les isomalto-oligosaccharides (IMO), les xylo-oligosaccharides (XOS), les arabino-xylo-oligosaccharides (AXOS), les mannan-oligosaccharides (MOS), les oligosaccharides de soja, glycosylsucrose (GS), lactosucrose (LS), lactulose (LA), palatinose-oligosaccharides (PAO), maltooligosaccharides, les amidons résistants, les gommes et/ou hydrolysats de celles-ci, pectines et/ou hydrolysats de celles-ci, ou des combinaisons de ceux-ci.

Dans des modes de réalisation, la composition comprend de plus une ou plusieurs vitamines.

Dans des modes de réalisation, les vitamines sont choisies parmi l'acide folique, la vitamine B12, la vitamine B6, vitamine A, la vitamine D, la vitamine E et la vitamine K.

La présente demande concerne aussi l'utilisation non-thérapeutique d'une composition telle que définie dans la présente description, pour maintenir ou accroître la masse et/ou la fonction musculaire chez un sujet en ayant besoin, en particulier chez un sujet dénutri, chez un sujet âgé, en particulier un sujet âgé dénutri, et chez un sujet pratiquant un exercice physique intense.

La présente demande est également relative à une composition telle que définie dans la présente description pour son utilisation en tant que médicament.

La présente demande a aussi trait à une composition telle que définie dans la présente description, pour son utilisation pour le traitement de la perte de masse et/ou de fonction musculaire chez un sujet en ayant besoin, en particulier chez un sujet choisi parmi les sujets en surpoids et les sujets obèses soumis à un régime alimentaire, les sujets cancéreux affectés d'une sarcopénie, les sujets diabétiques soumis à un régime alimentaire.

La présente demande est aussi relative à une composition telle que définie dans la présente description, pour son utilisation pour réduire l'insulino-résistance chez un sujet en ayant besoin, en particulier chez un sujet diabétique, et plus particulièrement un sujet affecté d'un diabète de type 2.

La présente description concerne aussi la souche *L. casei* SGC-K déposée à la Collection Nationale de Cultures de Microorganismes de l'Institut Pasteur le 8 mars 2021 sous le n° CNCM I-5663.

### Brève description des dessins

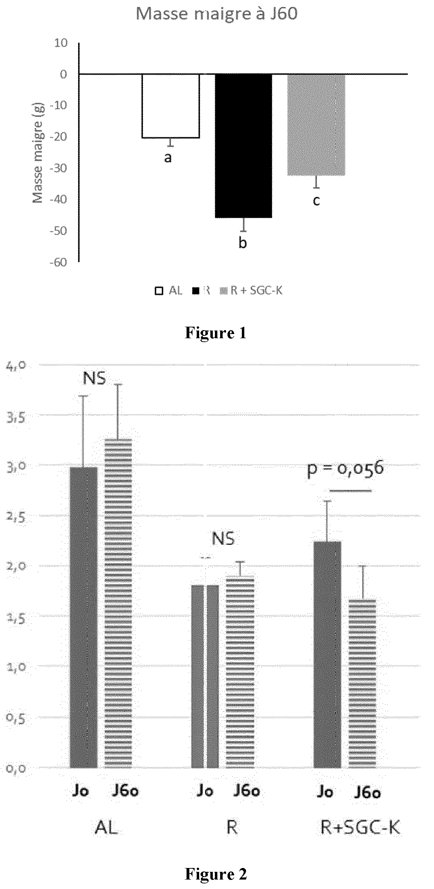

La **Figure 1** représente la perte de masse maigre à J 58, à la fin du protocole d'essai de 60 jours. En abscisse, les groupes d'animaux testés, respectivement (i) « AL » groupe d'animaux témoin nourris *ad libitum* en blanc, (ii) « R », groupe d'animaux à nutrition restreinte ayant reçu le tampon PBS témoin sans probiotique en noir, (iii) « R+ SGC-K » : groupe d'animaux à nutrition restreinte ayant reçu le probiotique *L. casei* SGC-K dans le tampon PBS en gris. En ordonnées, la perte de masse maigre, exprimées en grammes. Un changement de lettre (a, b, c) indique une différence significative entre les groupes. Ainsi, la différence est statistiquement significative (p<0.05 - n=59) respectivement, (i) entre les groupes « a » et «b », (ii) entre les groupes « a » et « c » et (ii) entre les groupes « b » et « c ».
La **Figure 2** représente l'état d'insulino-sensibilité des animaux illustré par les résultats du calcul de l'HOMA-IR. En ordonnées, chaque groupe de deux barres illustre les résultats à J0 (barre de gauche en noir) et à J60 (barre de droite en rayé), respectivement pour les groupes d'animaux suivants (n=53) : (i) « AL » groupe d'animaux témoin nourris *ad libitum,* (ii) « R », groupe d'animaux à nutrition restreinte ayant reçu le tampon PBS témoin sans probiotique, (iii) « R+ SGC-K » : groupe d'animaux à nutrition restreinte ayant reçu le probiotique *L. casei* SGC-K dans le tampon PBS.

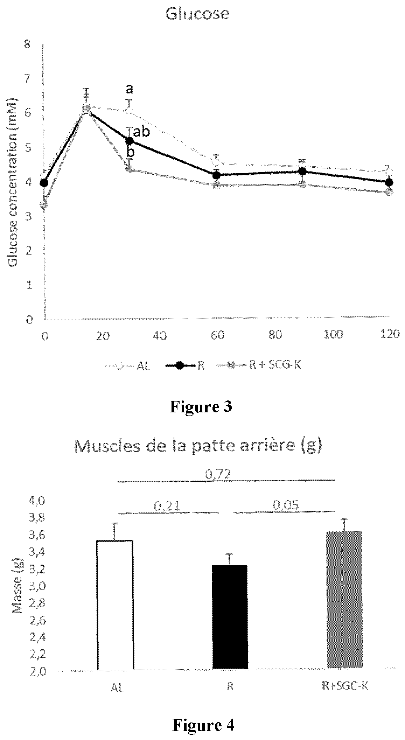

La **Figure 3** représente l'état d'insulino-sensibilité des animaux illustré par les résultats de glycémie suite à un test oral de glucose (OGTT - Oral glucose tolerance test) après 60 jours de restriction et traitement au probiotiques SGC-K. La courbe supérieure (cercles blancs) illustre les résultats obtenus pour le groupe d'animaux « AL », qui est le groupe d'animaux témoin nourris *ad libitum.* La courbe médiane (cercles noirs) illustre les résultats obtenus pour le groupe d'animaux « R », qui est le groupe d'animaux à nutrition restreinte ayant reçu le tampon PBS témoin. La courbe inférieure (cercles gris) illustre les résultats obtenus pour le groupe d'animaux « R+ SGC-K », qui est le groupe d'animaux à nutrition restreinte ayant reçu le probiotique *L. casei* SGC-K dans le tampon PBS. Un changement de lettre (a, b, c) indique une différence significative (p<0.05 - n=53).
La **Figure 4** représente la masse (en grammes) de la somme des muscles de la patte arrière des rats obtenus après dissection de la patte (muscles gastrocnémien, Soleus, Tibialis antérior et Extensor digitorum longus) suite à 30 jours de restriction alimentaire et supplémentation avec le probiotique *L. casei* SGC-K. En abscisse, les groupes d'animaux testés (n=61), respectivement (i) « AL » groupe d'animaux témoin nourris *ad libitum* en blanc, (ii) « R », groupe d'animaux à nutrition restreinte ayant reçu le tampon PBS témoin sans probiotique en noir, (iii) « R+ SGC-K » : groupe d'animaux à nutrition restreinte ayant reçu le probiotique *L. casei* SGC-K dans le tampon PBS en gris. En ordonnées, la masse musculaire, exprimées en grammes. La différence est statistiquement significative (p<0.05-n=61) entre les groupes R et R+SCG-K. la différence n'est pas significative entre les groupes AL et R (P=0.21) et entre les groupes AL et R+SCG-K (P=0.72).

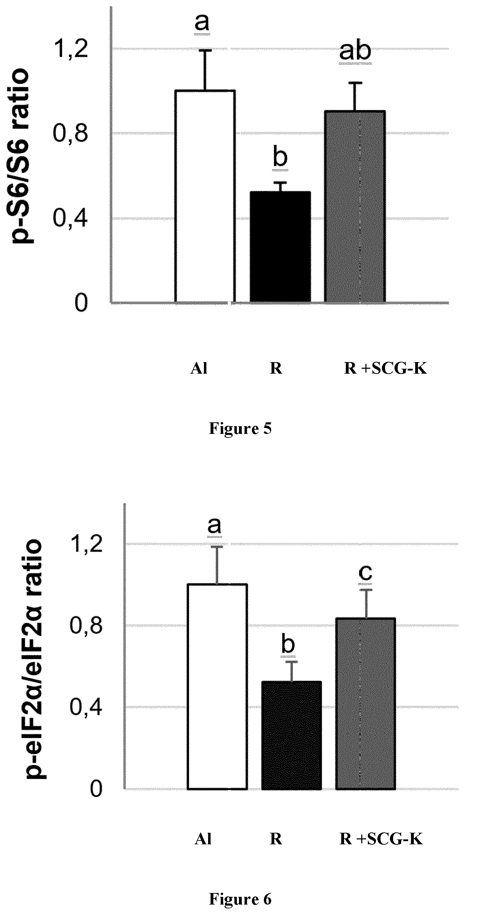

La **Figure 5** représente le niveau de phosphorylation de la protéine S6 dans un modèle de rat âgé suite à 30 jours de restriction alimentaire et supplémentation avec le probiotique *L. casei* SGC-K, respectivement (a) chez des animaux nourris *ad libitum ,* (b) chez des animaux soumis à une restriction alimentaire et (ab) chez des animaux soumis à une restriction alimentaire et supplémentés en souche SGCK. En ordonnées, les valeurs du rapport protéine S6 phosphorylée (p-S6)/ protéine S6 non phosphorylée (S6), soit le rapport « p-S6/S6 ». Les résultats sont ceux du rapport des densités de p-S6 et de S6 dans un essai de Western blot.
La **Figure 6** représente le niveau de phosphorylation du facteur d'élongation elF2a dans un modèle de rat âgé suite à 30 jours de restriction alimentaire et supplémentation avec le probiotique *L. casei* SGC-K, respectivement (a) chez des animaux nourris *ad libitum , (b)* chez des animaux soumis à une restriction alimentaire et (c) chez des animaux soumis à une restriction alimentaire et supplémentés en souche SGCK. En ordonnées, les valeurs du rapport protéine alF2a phosphorylée (p-elF2a)/ protéine elF2a non phosphorylée (elF2a), soit le rapport « p-elF2a/elF2a ». Les résultats sont ceux du rapport des densités de p-elF2a et de elF2a dans un essai de Western blot.

### Description détaillée

Il a été identifié selon la présente description une souche de *L. casei* qui possède une pluralité de propriétés bénéfiques pour la santé de sujets affectés d'une dénutrition.

La souche de *L. casei* selon la présente description consiste en la souche de *L. casei* SGC-K déposée le 8 mars 2021 auprès de la Collection Nationale de Cultures de Microorganismes de l'Institut Pasteur sous le n° CNCM I-5663. Cette souche peut être aussi dénommée « *L. casei* SGC-K » ou « SGC-K » dans la présente description.

Comme cela est montré dans les exemples, sur un modèle de sujet dénutri, l'administration orale de la souche *L. casei* SGC-K entraîne une réduction de la perte de masse musculaire chez ces sujets affectés d'une dénutrition, par comparaison avec les sujets dénutris n'ayant pas reçu la souche SGC-K. Plus particulièrement, la souche *L. casei* SGC-K entraîne une réduction de la perte de masse maigre musculaire chez ces sujets affectés d'une dénutrition, par comparaison avec les sujets dénutris n'ayant pas reçu la souche SGC-K.

De plus, les résultats rapportés dans les exemples montrent que l'administration orale de la souche *L. casei* SGC-K permet une diminution significative de la glycémie chez les sujets dénutris, par comparaison aux sujets n'ayant pas reçu la souche SGC-K. Ces résultats expérimentaux montrent que l'administration de la souche SGC-K provoque un effet insulino-sensibilisateur ; dont il peut -être tiré bénéfice chez des sujets en ayant besoin, en particulier chez des sujets affectés d'une insulino-résistance.

Les résultats rapportés dans les exemples montrent également que l'administration de la souche SGC-K induit une stimulation de l'expression de biomarqueurs impliqués dans la signalisation intracellulaire de l'insuline, comme la protéine S6 phosphorylée ou encore le facteur d'élongation elF2a phosphorylé, comme cela est visualisé par un accroissement des rapports respectifs p-S6/S6 et p-elF2a/elF2a, dans lesquels (1) « p-S6 » désigne la protéine S6 phosphorylée et « S6 » désigne la protéine S6 non phosphorylée et (2) « p-elF2a » désigne le facteur d'élongation elF2a phosphorylé et « elF2a » désigne le facteur d'élongation elF2a non phosphorylé.

Par protéine « S6 », on entend selon l'invention la protéine ribosomale S6 bien connue, qui est un composant de la sous-unité 40S du ribosome impliqué dans la traduction.

Par facteur d'élongation « elF2a », on entend selon l'invention la protéine bien connue qui est un facteur d'initiation de la traduction, nécessaire à la synthèse protéique .

Comme cela est connu, la survenue d'une insulino-résistance peut être un marqueur de dérèglements métaboliques, tels que le syndrome métabolique, lesquels dérèglements métaboliques représentent un facteur de risque de développer des pathologies. Ainsi, ces dérèglements métaboliques peuvent par exemple conduire à la survenue d'un diabète de type 2, favoriser une obésité, et être un élément prédisposant à des pathologies cardiovasculaires tel que le risque coronarien, certaines hépatopathies non alcooliques (stéatose hépatique), une hypertension ; le syndrome des ovaires polykystiques (SOPK), des pathologies chroniques intestinales, des pathologies chroniques intestinales rénales ou encore le syndrome métabolique.

Comme cela est connu, le syndrome métabolique est défini par la présence de valeurs considérées comme étant anomales pour au moins trois des cinq critères suivants : : (i) tour de taille, (ii) taux de triglycérides, (iii) taux de cholestérol HDL ; (iv) pression artérielle et (v) valeur de glycémie à jeun. Les valeurs considérées comme étant anormales peuvent différer selon le sexe du sujet, ces valeurs pouvant de plus être ethnocentrées (Voir par exemple Alberti et al., 2005, « The metabolic syndrome - a new worldwide definition » ; doi: 10.1016/S0140-6736(05)67402-8). A titre illustratif, l'occurrence d'un syndrome métabolique peut être diagnostiqué si au moins trois critères sont vérifiés, parmi les cinq critères suivants : (i) un tour de taille supérieur à 102 cm chez les hommes et supérieur à 88 cm chez les femmes, (ii) un taux de triglycérides supérieur à 150 mg/dL (1,7 mmol/L), ou le suivi d'un traitement spécifique contre un excès de triglycérides, (iii) un taux de cholestérol HDL inférieur ou égal à 40 mg/dL (1,03 mmol/L) chez les hommes ou inférieur ou égal à 50 mg/dL (1,29 mmol/L) chez les femmes, ou le suivi d'un traitement contre cette anomalie lipidique, (iv) une valeur de tension artérielle systolique supérieure à 130 mm Hg ou diastolique supérieure à 85 mm Hg, ou le suivi d'un traitement contre l'hypertension, ou (v) une valeur de glycémie à jeun supérieure ou égale à 100 mg/dL (5,6 mmol/L) ou la présence d'un diabète de type 2.

Comme cela est également connu, des situations inflammatoires sont liées à l'insulino-résistance. L'administration de la souche SGC-K est donc également utile pour la prévention ou le traitement de pathologies inflammatoires telles que (i) le syndrome ou la maladie du côlon irritable (ou « IBS/IBD » pour « Irritable Bowel Syndrome »/ »Irritable Bowel Disease »), (ii) la bronchopneumopathie chronique obstructive (ou « COPD » pour « Chronic Obstructive Pulmonary Disease », ou encore (iii) d'autres pathologies liées à une inflammation chronique, y compris des inflammations chroniques intestinales ou rénales, comme l'inflammation à long terme provoquée par une infection virale telle qu'une infection par un virus SARS-COV-2.

La présente description concerne la souche *L. casei* SGC-K déposée à la Collection Nationale de Cultures de Microorganismes de l'Institut Pasteur le 8 mars 2021 sous le n° CNCM I-5663 (aussi appelée souche « *L. casei* SGC-K » ou « SGC-K »).

Au sens de la présente description, la souche SGC-K est un probiotique.

Tel qu'il est utilisé ici, le terme "probiotique" désigne des micro-organismes vivants, lesquels, inclus en quantité suffisante, exercent un effet positif sur la physiologie, le confort et le bien-être.

### A. Compositions

La présente description concerne aussi une composition comprenant la souche SGC-K.

Comme cela apparaîtra dans la description, une composition comprenant la souche SGC-K est principalement destinée à des sujets dénutris, y compris à des sujets dénutris affectés d'un diabète de type 2.

Par « sujet dénutri », on entend selon la description un sujet présentant un état de dénutrition. L'état de dénutrition est bien connu de l'homme du métier. Les caractéristiques d'un état de dénutrition et ses conséquences sont documentés dans l'introduction de la présente description (voir aussi « Diagnostic de la dénutrition de l'enfant et de l'adulte - Méthode Recommandations pour la pratique clinique », Novembre 2019, Haute Autorité de Santé et Fédération Française de Nutrition, pages 11-12). Les sujets dénutris englobent les humains et les animaux non-humains, y compris les mammifères non-humains. Les sujets dénutris englobent donc les animaux de rente, les animaux d'élevage et les animaux de compagnie. Les sujets dénutris englobent les animaux de basse-cour (tels que poules, dindes, canards, oies, pigeons, cailles, faisans, autruches, émeus, nandous et pintades). Les sujets dénutris englobent aussi les animaux de pacage, tels que les équins (comme les chevaux, les ânes, les mulets, les bardots), les porcins, les bovins (comme les vaches, les buffles, les zébus, les bisons, les aurochs, les yacks), les ovins, les caprins, les camélidés (comme les chameaux, les dromadaires, les lamas, les alpagas) et les cervidés (comme les rennes, les cerfs). Les sujets dénutris englobent aussi les animaux de compagnie tels que les félins et les canins.

Les sujets dénutris englobent les humains, y compris les enfants et les personnes âgées dénutries.

La présente description concerne une composition nutritionnelle comprenant la souche SGC-K, qui est destinée à des sujets dénutris, affectés d'une perte de masse musculaire, lesdits sujets ne présentant pas de pathologie envers laquelle ladite composition nutritionnelle possède un effet prophylactique et/ou thérapeutique.

A titre d'exemple, un sujet sportif chez lequel il est nécessaire de maintenir ou d'augmenter la masse musculaire, ne présente pas de pathologie et ne nécessite qu'un apport de nature nutritionnelle. Selon un autre exemple, la composition selon la présente description peut être une composition nutritionnelle destinée à des personnes âgées dénutries, à des personnes en surpoids, dénutries par exemple du fait de la pratique d'un régime alimentaire.

Par « sujet âgé », au sens de la présente description, on entend un sujet humain ou un sujet mammifère non humain, y compris un animal de compagnie tel que le chien ou le chat, qui présente des signes de sénescence tels qu'une altération de fonctions métaboliques (par exemple absorption, digestion, excrétion), de difficultés locomotrices et d'une résistance réduite aux agressions extérieures.

Par « sujet âgé », ou « personne âgée », s'agissant d'un humain, on entend un sujet ayant 65 ans ou plus.

Par « sujet âgé », s'agissant d'un canin, en particulier d'un chien, on entend un sujet ayant (i) plus de 12 ans pour un canin, en particulier d'un chien, de petite taille ou (ii) plus de 9 ans pour un canin, en particulier d'un chien, de taille moyenne, ou (iii) plus de 7 ans, pour un canin, en particulier d'un chien, de grande taille.

Par « sujet âgé », s'agissant d'un félin, en particulier d'un chat, on entend un sujet ayant plus de 13 ans.

La présente description concerne aussi une composition pharmaceutique comprenant la souche SGC-K, qui est destinée à des sujets présentant, ou susceptibles de présenter, une pathologie sur laquelle ladite composition possède un effet prophylactique ou thérapeutique. A titre d'exemple, une composition selon la présente description consiste en une composition pharmaceutique lorsqu'elle est destinée à prévenir ou traiter une situation de résistance à l'insuline chez un sujet, comme par exemple chez un sujet affecté d'un diabète de type 2.

Toutefois, sauf si cela est précisé spécifiquement, une composition comprenant la souche SGC-K ne diffère pas dans ses caractéristiques générales exposées dans la description, selon qu'il s'agit d'une composition nutritionnelle ou d'une composition thérapeutique. Ainsi, pour l'essentiel, une composition pharmaceutique se distingue d'une composition nutritionnelle par le fait que la composition pharmaceutique possède, chez le sujet auquel elle est administrée, un effet de prévention et/ou un effet de traitement d'une maladie, spécifiquement un effet de sensibilisation à l'insuline chez un sujet insulinorésistant.

Dans certains modes de réalisation d'une composition selon la description, la souche SGC-K est utilisée comme la seule souche bactérienne présente dans la composition.

Dans certains autres modes de réalisation d'une composition selon la description, les bactéries de la souche SGC-K est combinée à des bactéries d'une ou plusieurs autres souches bactériennes probiotiques, ce qui englobe des souches bactériennes probiotiques commensales.

Des exemples non limitatifs de probiotiques englobent les souches de bactéries appartenant aux genres suivants *: Bifidobacterium, Lactobacillus, Lactococcus, Enterococcus, Streptococcus, Kluyveromyces, Saccharomyces, Candida* et leurs combinaisons.

Les probiotiques peuvent être choisis dans le groupe constitué par les espèces bactériennes suivantes : *Bifidobacterium longum, Bifidobacterium lactis, Bifidobacterium breve, Bifidobacterium infantis, Bifidobacterium adolescentis, Lactobacillus acidophilus, Lactobacillus casei (désormais Lacticaseibacillus casei), Lactobacillus paracasei* (désormais *Lacticaseibacillus paracasei), Lactobacillus salivarius* (désormais *Ligilactobacillus salivarius), Lactobacillus lactis* (désormais *Lactobacillus delbrueckii subsp. Lactis), Lactobacillus rhamnosus* (désormais *Lacticaseibacillus rhamnosus), Lactobacillus johnsonii, Lactobacillus plantarum* (désormais *Lactiplantibacillus plantarum subsp. Plantarum), Lactococcus lactis, Enterococcus faecium*, *Enterococcus faecalis*, *Saccharomyces cerevisiae, Saccharomyces boulardii* ou leurs mélanges, de préférence choisis dans le groupe constitué par *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM I- 3446), *Bifidobacterium breve* souche A, *Lactobacillus paracasei* NCC2461 (CNCM 1-2116), *Lactobacillus johnsonsii* NCC533 (CNCM 1-1225), *Lactobacillus rhamnosus* GG (ATCC53103), *Lactobacillus rhamnosus* NCC4007 (CGMCC 1. 3724), *Enterococcus faecium* SF 68 (NCC2768 ; NCIMB10415), *Faecalibacterium prausnitzii* et leurs combinaisons.

Dans certains modes de réalisation, la composition comprend aussi un ou plusieurs prébiotiques. Le terme « prébiotique » est utilisé dans son sens conventionnel dans l'état de la technique. Les prébiotiques consistent en des substances alimentaires favorisant la croissance de bactéries probiotiques, y compris les bactéries comprises dans le microbiote.

Des exemples non limitatifs de prébiotiques comprennent : les oligosaccharides contenant éventuellement du fructose, du galactose, du mannose ; les fibres alimentaires, en particulier les fibres fermentescibles, les fibres de soja ; l'inuline ; et leurs combinaisons. Les prébiotiques préférés sont les fructo-oligosaccharides (FOS), les galacto-oligosaccharides (GOS), les isomalto-oligosaccharides (IMO), les xylo-oligosaccharides (XOS), les arabino-xylo-oligosaccharides (AXOS), les mannan-oligosaccharides (MOS), les oligosaccharides de soja, glycosylsucrose (GS), lactosucrose (LS), lactulose (LA), palatinose-oligosaccharides (PAO), malto-oligosaccharides, les amidons résistants, les gommes et/ou hydrolysats de celles-ci, pectines et/ou hydrolysats de celles-ci, ou des combinaisons de ceux-ci.

Dans certains modes de réalisation, la composition comprend de plus une ou plusieurs vitamines. Les vitamines peuvent être l'acide folique, la vitamine B12 et la vitamine B6, en particulier l'acide folique et la vitamine B12, en particulier l'acide folique. Dans certains modes de réalisation, la composition comprend une ou plusieurs vitamines qui sont liposolubles, par exemple une ou plusieurs vitamines parmi la vitamine A, la vitamine D, la vitamine E et la vitamine K.

Dans certains modes de réalisation, la composition comprend un ou plusieurs polyphénols, tels que les flavanols, les flavanones, les flavonols, les acides hydroxycinnamiques et les anthocyanes.

Dans certains modes de réalisation, la composition comprend en outre un ou plusieurs minéraux. Les minéraux peuvent être choisis parmi le sodium, le potassium, le chlorure, le calcium, le phosphate, le magnésium, le fer, le zinc, le cuivre, le sélénium, le manganèse, le fluor, l'iode, le chrome ou le molybdène. Les minéraux sont généralement ajoutés sous forme de sel. Les minéraux peuvent être ajoutés seuls ou en combinaison.

Dans certains modes de réalisation, une composition selon la présente description comprend généralement des supports ou des véhicules. Les "supports" ou "véhicules" désignent des matériaux adaptés à l'administration et comprennent tout matériau connu dans l'état de la technique, tel que, par exemple, tout liquide, gel, solvant, diluant liquide, agent solubilisant ou autre, qui est non toxique et qui n'interagit pas avec les composants de la composition de manière délétère. Des exemples de supports nutritionnellement acceptables comprennent, par exemple, l'eau, les solutions salines, les alcools, les silicones, les cires, la vaseline, les huiles végétales, les polyéthylèneglycols, le propylèneglycol, les liposomes, les sucres, la gélatine, le lactose, l'amylose, le stéarate de magnésium, le talc, les agents tensioactifs, l'acide silicique, la paraffine visqueuse, l'huile de parfum, les monoglycérides et diglycérides d'acides gras, les esters d'acides gras de pétrole, l'hydroxyméthylcellulose, la polyvinylpyrrolidone, etc..

Dans certains modes de réalisation, la composition comprend de plus tout autre ingrédient ou excipient connu pour être employé dans le type de composition en question. Des exemples non limitatifs de tels ingrédients comprennent : des protéines, des acides aminés, des glucides, des oligosaccharides, des lipides, des nucléotides, des nucléosides, d'autres vitamines, des minéraux et d'autres micronutriments.

Dans certains modes de réalisation, la composition contient une source de glucides, par exemple sous forme des prébiotiques, ou s des prébiotiques lorsqu'ils sont présents dans la composition. Toute source de glucides que l'on trouve habituellement dans les préparations pour nourrissons, comme le lactose, le saccharose, la maltodextrine, l'amidon et leurs mélanges, peut être utilisée, bien que la source préférée de glucides soit le lactose.

Dans certains modes de réalisation, une composition selon la présente description consiste en une composition nutritionnelle.

Dans certains modes de réalisation, la composition nutritionnelle est choisie parmi les compositions alimentaires complètes, les compléments alimentaires, les compositions nutraceutiques et autres. La composition de la présente description peut être utilisée en tant qu'ingrédient alimentaire et/ou ingrédient d'aliments pour animaux. L'ingrédient alimentaire peut se présenter sous la forme d'une solution ou d'un solide, selon l'utilisation et/ou le mode d'application et/ou le mode d'administration. Tel qu'il est utilisé ici, le terme "aliment" fait référence à des compositions diététiques liquides (c'est-à-dire des boissons), solides ou semi-solides, en particulier des compositions alimentaires totales (remplacement d'aliments), qui ne nécessitent pas d'apport supplémentaire de nutriments ou des compositions de compléments alimentaires. Les compositions de compléments alimentaires ne remplacent pas complètement l'apport de nutriments par d'autres moyens. Tel qu'utilisé dans la présente description, le terme "ingrédient alimentaire" englobe une formulation qui est ou peut être ajoutée à des aliments fonctionnels ou à des denrées alimentaires en tant que complément alimentaire. Par "aliment nutritionnel" ou "nutraceutique" ou "fonctionnel", on entend une matière alimentaire qui contient des ingrédients ayant des effets bénéfiques sur la santé ou capables d'améliorer les fonctions physiologiques. Par "complément alimentaire", on entend une matière alimentaire ayant pour finalité de compléter un régime alimentaire normal. Un complément alimentaire est une source concentrée de nutriments ou d'autres substances ayant un effet nutritionnel ou physiologique, lorsqu'ils sont pris seuls ou en combinaison en petites quantités. Selon la présente description, "aliment fonctionnel" est utilisé pour désigner les matières alimentaires et les produits correspondants auxquels on attribue de l'importance non seulement en raison de leur valeur nutritionnelle et gustative mais aussi en raison de la présence d'ingrédients ayant des effets physiologiques bénéfiques.

Dans certains modes de réalisation, la composition est un produit laitier fermenté ou un produit à base de lait, qui est de préférence administrée ou ingérée par voie orale une ou plusieurs fois par jour. Les produits laitiers fermentés comprennent les produits à base de lait, tels que (mais sans s'y limiter) les desserts, les yaourts, les boissons au yaourt, le fromage blanc, le kéfir, les boissons à base de lait fermenté, le babeurre, les fromages, les vinaigrettes, les pâtes à tartiner à faible teneur en matières grasses, le fromage frais, les boissons à base de soja, la crème glacée, etc.

En variante, dans certains modes de réalisation, les compositions nutritionnelles et/ou de compléments nutritionnels peuvent être des produits non laitiers ou laitiers non fermentés. Les produits laitiers non fermentés peuvent inclure les crèmes glacées, les barres nutritionnelles et les assaisonnements, et autres. Les produits non laitiers peuvent comprendre des boissons en poudre et des barres nutritionnelles, etc. Les produits peuvent être fabriqués en utilisant des méthodes connues, telles que l'ajout d'une quantité efficace d'une bactérie SGC-K, ou d'une combinaison de bactéries incluant la souche SGC-K, à une base alimentaire, telle que du lait écrémé ou du lait ou une composition à base de lait, et réaliser une fermentation selon toute technique connue. Dans certains modes de réalisation, la composition est une boisson qui peut être une boisson fonctionnelle ou une boisson thérapeutique, une boisson désaltérante ou une boisson conventionnelle. À titre d'exemple, la composition selon la présente description peut être utilisée comme ingrédient pour des boissons gazeuses, un jus de fruit ou une boisson comprenant des protéines de lactosérum, des thés, des boissons au cacao, des boissons au lait, des yaourts y compris des yaourts à boire, des fromages, des crèmes glacées, des glaces à l'eau et des desserts, des confiseries, des biscuits, des gâteaux et des préparations pour gâteaux, des snacks, des aliments et boissons équilibrés, des glaçages, boisson de soja/jus acidifié, boisson au chocolat aseptique/rapportée, mélanges pour barres, préparations en poudre pour boissons, lait de soja et chocolat enrichi en calcium, boisson au café enrichie en calcium.

Dans certains modes de réalisation, la composition comprend tout autre ingrédient ou excipient connu pour être employé dans le type de composition en question. Des exemples non limitatifs de tels ingrédients comprennent : des protéines, des acides aminés, des glucides, des oligosaccharides, des lipides, des prébiotiques ou des probiotiques, des nucléotides, des nucléosides, d'autres vitamines, des minéraux et d'autres micronutriments.

Dans certains autres modes de réalisation, des bactéries de la souche SGC-K, le cas échéant en combinaison avec des bactéries probiotiques d'une ou plusieurs autres souches, sont administrées au sujet sous la forme d'une composition pharmaceutique, ce qui pourrait correspondre à un produit de type Live Biotherapeutic product (LBP) reference : Front Med (Lausanne) Rouanet et al2020 Jun 19;7:237. doi: 10.3389/fmed.2020.00237 . Par exemple, les bactéries d'intérêt peuvent être combinées avec des excipients pharmaceutiquement acceptables, et éventuellement des matrices à libération prolongée, telles que des polymères biodégradables, pour former des compositions thérapeutiques. Les termes "pharmaceutiquement" ou "pharmaceutiquement acceptable" désignent des entités moléculaires et des compositions qui ne produisent pas de réaction indésirable, de réaction allergique ou autre lorsqu'elles sont administrées à un mammifère, en particulier à l'homme, selon le cas. Un support ou excipient pharmaceutiquement acceptable désigne une charge, un diluant, un matériau d'encapsulation ou un auxiliaire de formulation non toxique, solide, semi-solide ou liquide, de n'importe quel type. Dans les compositions pharmaceutiques de la présente description pour une administration orale, sublinguale, sous-cutanée, intramusculaire, intraveineuse, transdermique, locale ou rectale, l'ingrédient actif ou la combinaison d'ingrédients actifs ingrédients actifs, peut être administré sous une forme d'administration unitaire, en mélange avec des supports pharmaceutiques classiques, à des animaux et des êtres humains. Les formes d'administration unitaires appropriées comprennent les formes d'administration par voie orale telles que les comprimés, les capsules de gel, les poudres, les granules et les suspensions ou solutions orales, les formes d'administration sublinguale et buccale, les aérosols, les implants, les formes d'administration sous-cutanée, transdermique, topique, intrapéritonéale, intramusculaire, intraveineuse, sous-cutanée, transdermique, intrathécale et intranasale et les formes d'administration rectale. Typiquement, les compositions pharmaceutiques contiennent des véhicules qui sont pharmaceutiquement acceptables pour une formulation susceptible d'être injectée. Il peut s'agir notamment de solutions salines isotoniques, stériles (phosphate monosodique ou disodique, chlorure de sodium, de potassium, de calcium ou de magnésium et similaires ou de mélanges de ces sels), ou de compositions sèches, notamment lyophilisées, qui par addition, selon le cas, d'eau stérilisée ou de sérum physiologique, permettent la constitution de solutions injectables. Les formes pharmaceutiques adaptées à l'usage injectable comprennent les solutions ou dispersions aqueuses stériles ; les formulations comprenant de l'huile de sésame, de l'huile d'arachide ou du propylène glycol aqueux ; et les poudres stériles pour la préparation extemporanée de solutions ou dispersions stériles injectables. Dans tous les cas, la forme doit être stérile et doit être fluide au point de pouvoir être facilement serrée.

La composition pharmaceutique doit être stable dans les conditions de fabrication et de stockage et doit être préservée de l'action contaminante des microorganismes, tels que les bactéries et les champignons. Les solutions comprenant les composés de la divulgation sous forme de base libre ou de sels pharmacologiquement acceptables peuvent être préparées dans de l'eau mélangée de manière appropriée avec un agent tensioactif, tel que l'hydroxypropylcellulose. Des dispersions peuvent également être préparées dans du glycérol, des polyéthylèneglycols liquides et leurs mélanges, ainsi que dans des huiles. Dans des conditions ordinaires de stockage et d'utilisation, ces préparations contiennent un conservateur pour empêcher la croissance des micro-organismes. L'au moins une bactérie SGC-K selon la présente description peut être formulée dans une composition sous une forme neutre ou sous forme de sel. Les sels pharmaceutiquement acceptables comprennent les sels d'addition d'acide (formés avec les groupes amino libres de la protéine) et qui sont formés avec des acides inorganiques tels que, par exemple, les acides chlorhydrique ou phosphorique, ou des acides organiques tels que les acides acétique, oxalique, tartrique, mandélique, et similaires. Les sels formés avec les groupes carboxyle libres peuvent également être dérivés de bases inorganiques telles que, par exemple, les hydroxydes de sodium, de potassium, d'ammonium, de calcium ou ferriques, et de bases organiques telles que l'isopropylamine, la triméthylamine, l'histidine, la procaïne et similaires.

Au sens de la présente description, le terme "quantité thérapeutiquement efficace" est une expression équivalente qui se réfère à la quantité d'une thérapie (par ex, un agent prophylactique ou thérapeutique), qui est suffisante pour réduire la gravité et/ou la durée d'une maladie, améliorer un ou plusieurs de ses symptômes, empêcher la progression d'une maladie ou provoquer la régression d'une maladie, ou qui est suffisante pour aboutir à la prévention du développement, de la récurrence, de l'apparition ou de la progression d'une maladie ou d'un ou plusieurs de ses symptômes, ou renforcer ou améliorer le ou les effets prophylactiques et/ou thérapeutiques d'une autre thérapie (par exemple, un autre agent thérapeutique) utile pour traiter une maladie. Typiquement, dans une composition pharmaceutique selon la présente description, les bactéries de la souche SGC-K sont présentes en une quantité suffisante pour induire une réduction de l'insulino-résistance chez le sujet traité. La mesure de l'insulino-résistance chez un sujet peut être réalisée selon toute technique connue de l'homme du métier. De manière préférée, la mesure de l'insulinorésistance chez un sujet est réalisée par le calcul de l'indice HOMA IR, comme illustré dans les exemples. La méthode HOMA IR (pour « HomeOstasis Model Assessement for Insulin Resistance »), qui a été développée à partir de la modélisation mathématique des réponses quantitatives des principaux organes du métabolisme du glucose. La valeur de l'indice HOMA IR est obtenue à l'aide d'une valeur plasmatique d'insuline ou de C-peptide et de glycémie à jeun (Sheen, 2007, Therapie, Vol. 62 : 311-318). La survenue et/ou le niveau d'insulino-résistance peut aussi être établi par la mesure de la glycémie à jeun, par la mesure de l'insulinémie à jeun ou encore par le test OGTT (pour « Oral Glucose Tolerance Test », ou « épreuve d'hyperglycémie provoquée » - Voir par exemple, « Mesure de l'insulinorésistance et de la tolérance au glucose », 2006, Haute Autorité de Santé (HAS), France).

Dans une composition selon la description, qu'il s'agisse d'une composition nutritionnelle ou alimentaire, le cas échéant d'un complément alimentaire, ou qu'il s'agisse d'une composition pharmaceutique, la ou les bactéries peuvent se présenter sous des formes variées, par exemple sous forme liquide ou sous forme de poudre. La ou les bactéries peuvent se présenter sous forme lyophilisée.

La quantité de bactéries de la souche SGC-K qui est apportée au sujet peut être variable, selon l'état physiologique dudit sujet, et notamment selon le niveau de déséquilibre apport en nutriments par rapport au besoin en nutriments dudit sujet. La quantité de bactéries SGC-K qui doit être apportée audit sujet peut être aisément adaptée par l'homme du métier.

Dans des modes de réalisation préférés, que la composition soit une composition nutritionnelle ou une composition pharmaceutique, la composition comprend une quantité de bactéries de la souche SGC-K qui est appropriée pour un apport quotidien, de préférence un apport oral quotidien, d'au moins 10³ unités formant colonies (ou « CFU » pour « Colony Forming Units »)

Dans ces modes de réalisation préférés, l'apport quotidien, en particulier l'apport oral quotidien, en bactéries de la souche SGC-K est au plus de 10¹³ unités formant colonies (ou « CFU » pour « Colony Forming Units »).

Dans les modes de réalisation dans lesquels la composition comprend de plus d'autres bactéries probiotiques, la quantité de ces bactéries probiotiques est déterminée par l'homme du métier sur la base de ses connaissances générales. La quantité des ces autres bactéries probiotiques peut varier de 10³ à 10¹³ autres bactéries probiotiques.

Dans certains modes de réalisation, une composition selon la présente description comprend généralement des supports ou des véhicules. Les "supports" ou "véhicules" désignent des matériaux adaptés à l'administration et comprennent tout matériau connu dans l'état de la technique, tel que, par exemple, tout liquide, gel, solvant, diluant liquide, agent solubilisant ou autre, qui est non toxique et qui n'interagit pas avec les composants de la composition de manière délétère. Des exemples de supports nutritionnellement acceptables comprennent, par exemple, l'eau, les solutions salines, les alcools, les silicones, les cires, la vaseline, les huiles végétales, les polyéthylèneglycols, le propylèneglycol, les liposomes, les sucres, la gélatine, le lactose, l'amylose, le stéarate de magnésium, le talc, les agents tensioactifs, l'acide silicique, la paraffine visqueuse, l'huile de parfum, les monoglycérides et diglycérides d'acides gras, les esters d'acides gras de pétrole, l'hydroxyméthylcellulose, la polyvinylpyrrolidone, etc..

Dans d'autres modes de réalisation d'une composition selon la présente description, ladite composition se présente sous la forme d'une composition pharmaceutique comprenant un ou plusieurs excipients pharmaceutiquement acceptables.

Une telle composition pharmaceutique peut être présentée sous la forme d'un emballage comprenant une pluralité d'unités de dosage.

Le terme « unité de dosage » est utilisé dans son sens conventionnel en pharmacie (p. ex. une pilule, une gélule, un comprimé, le contenu d'une ampoule, etc.).

### B. Utilisations

La présente description concerne les utilisations nutritionnelles et les utilisations thérapeutiques d'une composition telle que définie dans la présente demande.

Les caractéristiques de composition, y compris d'une composition nutritionnelle et d'une composition thérapeutique, y compris dans divers modes de réalisation, sont décrites en détail dans la présente description, ce qui inclut les quantités d'ingrédient(s) actif(s), spécialement les quantités de bactéries de la souche SGC-K comprises dans ces compositions.

La présente description concerne l'utilisation d'une composition telle que définie dans la présente demande pour maintenir ou accroître la masse musculaire et/ou la fonction musculaire chez un sujet en ayant besoin.

Dans de nombreux modes de réalisation, une composition selon la présente description consiste en une composition nutritionnelle, c'est-à-dire en une composition qui est utilisée à des fins nutritionnelles pour maintenir ou accroître la masse musculaire et/ou la fonction musculaire chez un sujet en ayant besoin. Une telle utilisation demeure une utilisation nutritionnelle de nature non thérapeutique, y compris chez certains sujets malades dont la pathologie dont ils sont affectés est concomitante avec une perte de masse musculaire et/ou de fonction musculaire. En effet, dans ces contextes, l'apport d'une composition nutritionnelle selon la présente description n'a pas pour effet de prévenir ou traiter la pathologie dont est affecté le sujet, mais a pour effet d'apporter un bénéfice nutritionnel audit sujet qui reçoit par ailleurs un traitement thérapeutique destiné à soigner ladite pathologie.

En particulier, l'apport d'une composition selon la présente description à un sujet ne présentant pas de trouble associé à une insulino-résistance, que ce sujet soit ou non affecté d'une pathologie, consiste en une utilisation nutritionnelle d'une telle composition.

Toutefois, les dispositions réglementaires peuvent varier selon les pays concernés, ce qui entraîne qu'une composition selon la présente description, dans ses modes d'utilisation à effets nutritionnels décrits ci-dessus, peut le cas échéant être considéré d'un point de vue règlementaire régissant le droit de la Santé, comme un médicament.

La présente description est relative à l'utilisation d'une composition nutritionnelle telle que définie dans la présente demande pour maintenir ou accroître la masse et/ou la fonction musculaire chez un sujet en ayant besoin. De préférence, les sujets ayant besoin d'une composition nutritionnelle telle que définie dans la présente demande sont choisis parmi les sujets dénutris, les sujets âgés, en particulier les sujets âgés dénutris, et les sujets pratiquant un exercice physique intense ou encore les sujets ayant des besoins protéiques accrus.

Dans certains modes de réalisation, ladite composition nutritionnelle consiste en une composition de complément nutritionnel tel que défini dans la présente description.

Dans certains modes de réalisation, une composition nutritionnelle conforme à la présente description, le cas échéant sous la forme d'une composition de complément alimentaire, est formulée pour une administration quotidienne, par exemple pour une administration quotidienne en une ou deux prises, par exemple au moment des repas.

De manière générale, une composition nutritionnelle selon la présente description peut être administrée au sujet jusqu'à ce que la valeur de masse musculaire et/ou de fonction musculaire atteigne un niveau prédéterminé, indicatif d'une masse musculaire normale (voir par exemple « Diagnostic de la dénutrition de l'enfant et de l'adulte - Méthode Recommandations pour la pratique clinique », Novembre 2019, Haute Autorité de Santé et Fédération Française de Nutrition, le tableau de la fin de la page 11). A titre illustratif, le niveau de la masse musculaire et/ou de fonction musculaire peut être déterminé au regard de l'un au moins des critères suivants : (i) force de préhension mesurée par un dynamomètre, par exemple en kg, (ii) vitesse de marche, par exemple mesurée en m/s, (iii) l'indice de surface musculaire en L3, par exemple mesurée en cm²/m², par exemple à l'aide d'une dispositif scanner tel qu'en IRM, (iv) l'indice de masse musculaire, par exemple en kg/m², par exemple mesuré par impédancemétrie, (v) indice de masse non grasse, par exemple en kg/m², par exemple mesuré par impédancemétrie), ou encore (vi) la masse musculaire appendiculaire (DEXA), par exemple mesuré en kg/m². La mesure de dynamique métabolique musculaire (qui a pour conséquence de réguler la masse et la fonction du muscle) peut aussi, dans certains cas, être évaluée en utilisant des techniques plus invasives, par exemple des techniques permettant de mesurer la vitesse de renouvellement des protéines par injection d'acides aminés marqués par des isotopes stables.

Dans certains modes de réalisation, une composition nutritionnelle conforme à la présente description est administrée au sujet de manière chronique, pendant une longue période de temps, par exemple pendant plusieurs mois ou même pendant plusieurs années, aux fins de maintenir le niveau de masse musculaire et/ou de fonction musculaire à une valeur prédéterminée, indicative de l'absence d'une masse musculaire normale et/ou d'une fonction musculaire normale.

La présente description concerne aussi des utilisations médicales d'une composition telle que décrite dans la présente demande.

La description est relative à une composition telle définie dans la présente demande pour son utilisation en tant que médicament.

Elle est relative à l'utilisation d'une composition telle que définie dans la présente demande pour la fabrication d'un médicament.

La description concerne aussi une composition telle que définie dans la présente demande pour son utilisation pour prévenir ou traiter une perte de masse musculaire chez des sujets en ayant besoin. De préférence, les sujets en ayant besoin sont choisis parmi les sujets en surpoids et les sujets obèses, souvent insulino-résistants, et soumis à un régime alimentaire hypocalorique et/ou hypoglucidique, les sujets obèses ou non ayant subi une intervention de chirurgie bariatrique, qui ont donc une diminution forte de leur apport énergétique global mais dont l'apport protéique doit être maintenu pour éviter une fonte de masse musculaire, les sujets cancéreux, en particulier les sujets cancéreux affectés d'une cachexie, les sujets traités par antibiothérapie, les sujets affectés par une infection virale ou bactérienne, les sujets diabétiques soumis à un régime alimentaire hypocalorique et/ou hypoglucidique, ou encore chez des sujets en réanimation qui sont nourris par voie entérale.

La description concerne aussi l'utilisation d'une composition telle que définie dans la présente demande pour la fabrication d'un médicament pour prévenir ou traiter une perte de masse musculaire chez des sujets en ayant besoin. De préférence, les sujets en ayant besoin sont choisis parmi les sujets en surpoids et les sujets obèses soumis à un régime alimentaire hypocalorique, les sujets obèses ou non ayant subi une intervention de chirurgie bariatrique les sujets cancéreux, en particulier les sujets cancéreux affectés d'une sarcopénie, les sujets traités par antibiothérapie, les sujets affectés par une infection virale ou bactérienne, les sujets affectés d'une sarcopénie, les sujets diabétiques soumis à un régime alimentaire hypocalorique, ou encore chez des sujets en réanimation qui sont nourris par voie entérale.

La description concerne aussi une méthode pour prévenir ou traiter une perte de masse et de fonction musculaire chez des sujets en ayant besoin comprenant l'administration audit sujet d'une composition telle que définie dans la présente demande.

Ainsi, dans certains modes de réalisation, une composition telle que définie dans la présente demande peut être utilisée à titre préventif. La composition peut par exemple être administrée, à titre préventif, à des sujets présentant un risque de développer une perte de masse et de fonction musculaire, ce qui englobe les sujets affectés d'une insulinorésistance, pour les sujets obèses suivant un traitement visant la perte de poids, pour lesquels une perte de masse musculaire et/ou une perte de fonction musculaire n'est pas survenue.

De manière générale, une composition thérapeutique selon la présente description peut être administrée au sujet jusqu'à ce que la valeur de masse musculaire et/ou de la fonction musculaire atteigne un niveau prédéterminé, indicatif d'une absence de sarcopénie

Dans certains modes de réalisation, une composition thérapeutique conforme à la présente description est administrée au sujet de manière chronique, pendant une longue période de temps, par exemple pendant plusieurs mois ou même pendant plusieurs années, aux fins de maintenir la valeur de masse musculaire et/ou de fonction musculaire à un niveau prédéterminé, indicatif de l'absence d'une sarcopénie.

Comme cela est montré dans les exemples, une composition conforme à la présente description, qui comprend au moins des bactéries de la souche SGC-K, possède des propriétés d'insulino-sensibilisation, c'est-à-dire possède la capacité à réduire une insulinorésistance chez un sujet, en particulier chez un sujet diabétique ou chez un sujet présentant une pathologie cardiovasculaire, en particulier une pathologie cardiovasculaire liée à un diabète.

La description est également relative à une d'une composition telle que définie dans la présente demande pour son utilisation pour réduire l'insulino-résistance chez un sujet en ayant besoin, en particulier chez un sujet diabétique ou chez un sujet présentant une pathologie cardiovasculaire, en particulier une pathologie cardiovasculaire liée à un diabète.

La description concerne l'utilisation d'une composition telle que définie dans la présente demande pour la fabrication d'un médicament pour réduire l'insulino-résistance chez un sujet en ayant besoin, en particulier chez un sujet diabétique, et plus particulièrement un sujet affecté d'un diabète de type 2 ou chez un sujet présentant une pathologie cardiovasculaire, en particulier une pathologie cardiovasculaire liée à un diabète.

La description concerne aussi une méthode pour réduire l'insulino-résistance chez un sujet en ayant besoin, en particulier chez un sujet diabétique, comprenant l'administration audit sujet d'une composition telle que définie dans la présente demande.

De manière générale, une composition thérapeutique selon la présente description peut être administrée au sujet jusqu'à ce que la valeur d'insulino-résistance, par exemple jusqu'à ce que son indice HOMA-IR, atteigne une valeur prédéterminée, indicative d'une insulino-résistance réduite.

De manière générale, une composition thérapeutique selon la présente description peut être administrée au sujet jusqu'à ce que la valeur de masse et de fonction musculaire par exemple jusqu'à ce que son indice HOMA-IR, atteigne une valeur prédéterminée, indicative d'une masse musculaire et /ou d'une fonction musculaire d'un sujet sain.

Dans certains modes de réalisation, une composition thérapeutique conforme à la présente description est administrée au sujet de manière chronique, pendant une longue période de temps, par exemple pendant plusieurs mois ou même pendant plusieurs années, aux fins de maintenir la valeur de masse et/ou de fonction musculaire, par exemple l'indice HOMA IR, à une valeur prédéterminée, indicative d'une insulino-résistance réduite.

### EXEMPLES

### Exemple 1 : Effet de la souche L. casei SGC-K sur des modèles animaux âgés ayant un apport alimentaire restreint

### A. Matériels et Méthodes

### A.1. Modèle et diète expérimentaux

Le modèle expérimental qui a été utilisé est le rat mâle Wistar (Charles River, *Écully),* âgé de 18-20 mois. Les animaux ont été élevés au cours de la période expérimentale en cycle nycthéméral inversé (nuit de 8h à 20h et jour de 20h à 8h). Les animaux ont été placés en cage individuelle deux semaines avant le début de l'expérimentation (avant le début de la restriction) puis mis en lot (15 animaux par lots) en homogénéisant leur poids, leur masse grasse et leur masse maigre.

Les rats âgés (mâles Wistar, 18-20 mois) ont été mis en restriction alimentaire pour mimer la fragilité du sujet âgé généralement associée à une sous-nutrition. Les apports protéiques des personnes âgés fragiles étant estimée, pour une majorité de cette population, à moins de 80% des recommandations (Sieber, 2019, Malnutrition and sarcopenia : Aging Clinical and Experimental research, Vol. 31(6) : 793-798), les animaux ont donc été restreints à 75% de l'*adlibitum* pour mimer une situation nutritionnelle similaire. Le groupe des animaux restreints (groupe R) a été comparé à un groupe pouvant manger à volonté (groupe AL) ainsi qu'à un groupe d'animaux restreints recevant le probiotique la souche L. *casei SGC-K* (groupe R+ *L. casei SGC-K).* Ces derniers ont été supplémentés quotidiennement en bactéries probiotiques (~5.10⁹ bactéries/jour) pendant 1 mois (expérimentation 30 jours) ou 2 mois (expérimentation 60 jours).

Le niveau d'alimentation étant contrôlé et similaire chez les animaux témoins restreints supplémentés ou non en probiotique *L. casei* SGC-K, l'effet du probiotique a été comparé relativement à des animaux restreints témoins, et relativement à des animaux à niveau d'ingestion normal.

### A.2. Supplémentation en probiotiques

Les bactéries probiotiques *L. casei* SGC-K utilisées ont été concentrées dans des tubes congelés à -20°C. Les bactéries probiotiques ont été données vivantes aux rats supplémentés (groupe R+SGCK) tous les matins, avant le repas quotidien, à raison d'une dose de 5x10⁹ bactéries par rat. La distribution des probiotiques (ou du tampon témoin sans probiotique) a été réalisée avant le repas quotidien afin de limiter le stress qui serait provoqué par le gavage.

Le culot bactérien était repris dans du tampon phosphate salin (PBS), et dispersé à la dose voulue de 5x10⁹ bactéries sur de la poudre de croquettes (1g). Pour les rats du groupe restreint (R), un volume équivalent de PBS a été déposé sur la poudre de croquettes, afin de limiter les éventuels biais d'expérience. Les bactéries sont ingérées par les rats en 15 minutes, puis le reste de l'aliment journalier est fourni. La survie des bactéries diluées dans le PBS, et sur les croquettes durant 30 minutes avait été préalablement vérifiée *in vitro.*

Deux expérimentations ont été réalisées : Une expérimentation sur 60 jours a permis l'obtention des résultats longitudinaux concernant la masse maigre (figure 1) et les paramètres d'insulino-sensibilité (figures 2 et 3). Une expérimentation plus courte d'une durée de 30 jours a permis de générer les données en point final sur la masse musculaire (figure 4) et les protéines musculaires (Figures 5 et 6).

### A.3. Suivi phénotypique longitudinal

### A.3.1. Suivi du poids

Les animaux ont été pesés trois fois par semaine durant toute la durée de l'expérimentation. Cela a notamment permis de surveiller que les rats ne perdaient pas plus de 20% de leur poids initial (critère éthique d'expérimentation animale), et à déterminer si la prise des probiotiques *L. casei* SGC-K avait un effet spécifique sur leur poids.

### A.3.2. Suivi de la composition corporelle - Expérimentation 60 jours

La composition corporelle a été évaluée par la méthode de l'EchoMRI (*Echo Medical Systems, Houston, TX USA*). Les mesures réalisées par cet appareil se basent sur les différences inhérentes aux propriétés de résonance magnétique nucléaire des atomes d'hydrogène et de la densité relative de l'hydrogène dans les fluides et les tissus pour obtenir des estimations de la masse grasse, de la masse maigre. Le rat vigile est placé dans un tube en plastique translucide et immobilisé. Le support est ensuite inséré dans un espace tubulaire sur le côté du système EchoMRI et la mesure est lancée. Cela permet une analyse noninvasive de la composition corporelle en moins de trois minutes sur des animaux vigiles. La composition corporelle des animaux a été mesurée à J-7, à J30 et à J56.

### A.3.3. Test de tolérance au glucose avant et après le traitement aux probiotiques - expérimentation 60 jours

Ce test consiste en un gavage des animaux à jeun à raison de 1g de glucose par kg de masse corporelle de rat et permet d'estimer la réponse insulinique et glycémique à ce bolus de glucose, donc le niveau d'insulino-sensibilité. Un prélèvement de sang périphérique (veine de la queue) a été réalisé à jeun. Puis des prélèvements de sang sont réalisés 15, 30, 60, 90 et 120 minutes après ingestion du bolus de glucose. Le plasma est obtenu après centrifugation à 1300 tours/min pendant 10 minutes. L'insuline a été dosée dans le plasma par ELISA (*Mercodia rat insulin ELISA*) et la glycémie par dosage enzymatique (*Glucose GOD-PAP, Biolab*). Les lectures ont été réalisées à l'aide du lecteur de plaques UV/visible Tecan Infinite 200 Pro (Tecan, France). A partir de l'insulinémie et de la glycémie à jeun, l'index d'insulino-résistance HOMA (*HOmeostasis Model Accessement of insulin resistance*) peut être calculé selon la formule suivante : (insulinémie à jeun (mU/L) x glycémie à jeun (mM))/22,5. Les OGTT ont été réalisés avant l'ingestion des probiotiques et 2 jours avant l'euthanasie des animaux à J60.

### A.4 Effet en point final sur la masse musculaire - expérimentation 30 jours

Cette mesure consiste à disséquer les muscles principaux de la patte arrière (gastrocnemien, tibialis antérieur, soléus et extensor digitorum longus) des rats, de les peser et de faire la somme des masses à la fin de la période expérimentale ;

### A.5. Analyses stastistiques

Toutes les analyses de calculs statistiques ont été effectuées à l'aide du logiciel « Sigma Plot ». Les données sont exprimées en tant que moyenne ± erreur standard à la moyenne (SEM).

Une analyse de variance (ANOVA) à deux facteurs en mesures répétées a été utilisée pour l'analyse en cinétique des données d'ingérés, de suivi de masse corporelle ainsi que pour les données des tests de tolérance au glucose (glycémie, insulinémie et HOMA). Une analyse de variance (ANOVA) à un facteur a été réalisée pour l'analyse des données en point final de la masse grasse et de la masse maigre (ou de leur évolution : valeur à J56 - J0), la masse des différents tissus à l'euthanasie ainsi que pour les concentrations en métabolites/hormones. Le test de Holm-Sidak a été utilisé pour des comparaisons post-hoc des moyennes. Des tests t de student one été réalisés sur les données de poids de muscles de la patte arrière en point final.

Dans tous les cas, l'hypothèse de l'existence de différences significatives entre les facteurs testés est validée pour un risque d'erreur inférieur à 5%. Une tendance est évoquée pour un risque d'erreur de 5 à 10%.

### B. Résultats

### B.1. Poids et composition corporelle (expérimentation 60 jours) (n=59)

La consommation des animaux a été contrôlée tout le long de l'expérimentation et montre que les animaux R, et R+SGC-K ingéraient en moyenne respectivement 21,3 ± 0,1 g et 20,9 ± 0,1 g d'aliment alors que les animaux AL, 28,9 ± 0,3 g. La restriction alimentaire s'est traduite par une diminution de la masse corporelle dès le septième jour de restriction comparativement aux animaux non restreints (effet traitement P<0,001; R, R+SGC-K vs AL). La supplémentation en probiotiques *L. casei* SGC-K (groupe R+SGC-K) n'as pas eu d'effet spécifique lors de la restriction. A la fin de l'étude (expérimentation 60 jours), la perte de masse corporelle moyenne des animaux R et R+SGC-K était de 13,6 ± 0,6%.

L'autre composante de la perte de masse corporelle est la perte de masse maigre. La perte de masse maigre est significativement plus importante avec la restriction chez les animaux R et R+SGC-K en comparaison des AL (P<0,05) (figure 1).

Ces résultats indiquent que l'ingestion du probiotique *L. casei* SGC-K permet de limiter la perte de masse maigre qui est provoquée par une sous-nutrition.

### B.2. Etat d'insulino-sensibilité (expérimentation 60 jours) (n=53)

L'état d'insulino-sensibilité des animaux a été évalué à J-7 et J58 à l'aide de l'index HOMA-IR. Entre J-7 et J58, la valeur de l'indice HOMA-IR (indice composite intégrant l'insulinémie et la glycémie) ne varie pas de façon significative dans le temps au sein de chaque lot d'animaux (figure 2). Lorsqu'on compare la valeur de l'index HOMA-IR entre les différents traitements à J58, on observe que la restriction alimentaire tend à diminuer l'HOMA-IR pour le groupe R+SGC-K par rapport aux AL (P=0,066 ; P=0,068 respectivement), sans être significative pour R.

Les résultats montrent que l'excursion de l'insulinémie est identique pour tous les animaux ayant une nutrition restreinte, ce qui englobe (i) le groupe R d'animaux témoins à nutrition restreinte et (ii) le groupe R+ d'animaux à nutrition restreinte ayant reçu le probiotique *L. casei* SGC-K.

A jeun, la valeur de l'indice HOMA-IR tend à diminuer au cours des deux mois de supplémentation avec le probiotique *L. casei* SGC-K (groupe R+).

L'analyse des données de l'OGTT à J58 montre que l'excursion glycémique du groupe R+SGC-K était réduite avec, 30 minutes après l'ingestion du bolus de glucose, une glycémie inférieure au lot AL (P = 0,015) (figure 3).

De plus, les résultats de la figure 3 montrent que la glycémie des animaux du groupe R+SGC-K ayant reçu le probiotique *L. casei* SGC-K diminue significativement plus rapidement, 30 minutes après l'ingestion du bolus, comparativement aux animaux du groupe R également à nutrition restreinte mais qui n'ont pas reçu de probiotique (P = 0,01) (figure 3).

L'ensemble de ces résultats suggère que le probiotique *L. casei* SGC-K provoque un effet insulino-sensibilisateur.

L'effet insulino-sensibilisateur qui est provoqué par l'ingestion du probiotique *L. casei* SGC-K pourrait contribuer à la préservation de la masse maigre musculaire.

### B.3 Effet sur la masse musculaire (expérimentation 30 jours) (n=61)

Ces résultats ont été obtenus sur une autre étude (sur 30 jours) que celle ayant permis le suivi longitudinal de l'évolution de masse maigre et de l'insulino-sensibilité (cette dernière réalisée sur 60 jours). La masse des quatre muscles d'une patte arrière des rats (gastrocnemien, tibialis antérieur, soléus et extensor digitorum longus) a été mesurée à la fin d'une période expérimentale de 30 jours. Les résultats montrent que le probiotique *L. casei* SGC-K a permis de maintenir la masse musculaire des rats en sous nutrition (groupe R+SGC-K et AL non différents, P= 0.72) avec en parallèle une masse musculaire significativement supérieure chez les rats du groupe R+SGC-K relativement au groupe R (+12%, P=0.05).

Ces données suggèrent que l'ingestion du probiotique *L. casei* SGC-K a permis la préservation de la masse musculaire, et ceci même dans un contexte de sous nutrition.

### Exemple 2 : Effet de la souche L. casei SGC-K sur la sensibilité à l'insuline chez des modèles animaux âgés ayant un apport alimentaire restreint

### A. Matériels et Méthodes

Les modèles animaux expérimentaux ont été réalisés comme décrit à l'exemple 1.

### A. Matériels et Méthodes des western blots (expérimentation 30 jours) (n=61)

Le Western blot permet la détection et la quantification relative des protéines S6 et eIF2α phosporylée (pS6 et p-eIF2α) ou non phosporylée (S6 et eIF2α). Le rapport de quantité de protéines phosphorylées/déphosphorylées de S6 et eIF2α illustre le niveau d'activation de la voie de signalisation mTOR. Les muscles gastrocnémiens (50mg) des animaux (protococle 30 jours) ont été congelés dans l'azote liquide et broyés pour les réduire en poudre. Les échantillons ont ensuite été homogénéisés dans du tampon d'extraction, centrifugés (10,000 g at 4 °C 10 min) pour récupérer les surnageants. Les surnageants sont dilués et gardés à -20 °C pour déterminer la concentration de protéines (Pierce ^{™} BCA Protein Assay Kit). 16µg de protéines sont déposées sur un gel dénaturant SDS-PAGE (Mini-PROTEAN TGX Precast Gels, BIO-RAD, France) et transférées sur une membrane PVDF (Trans-Blot^{®} Turbo^{™} Midi-size PVDF, BIO-RAD, France). La membrane a été préhybridée avec 5% de BSA (bovine serum albumin). Les anticorps primaires sont lapin p-S6, S235/236 (Cell signaling Technology 2211S), lapin S6 (Cell signaling Technology 2217S), lapin p-eIF2α S51 (abcam 32157), lapin eiF2α (abcam 242148) dilués 1: 1000 or 1: 2000. Les anticorps secondaires sont anti-lapin HRP-linked antibodies (Cell signaling Technology 7074) dilués 1: 2000. Après préhybridation, hybridation et lavages, les signaux des bandes ont été quantifiés avec le logiciel GeneTools (Syngene).

### B. RESULTATS

Comme dans le cas de l'organisme entier, il est montré dans l'Exemple 2 que des protéines impliquées dans la signalisation intracellulaire de l'insuline, comme la protéine S6 et le facteur d'élongation elF2a, étaient stimulées par la souche bactérienne SGCK (en situation post prandiale) dans le muscle chez le modèle de rats âgés fragiles (en sous nutrition chronique).

Après l'ingestion du repas, (i) un médiateur intracellulaire de l'action de l'insuline, à savoir la phosphorylation de la protéine S6, représenté sur la Figure 5 et (ii) la phosphorylation du facteur d'élongation eIF2a impliqué dans la stimulation de la synthèse protéique, également sensible à l'insuline, représenté sur la Figure 6, ont été mesurés.

Les résultats représentés sur chacune des figures 5 et 6 montrent que ces deux indicateurs de la sensibilité à l'insuline ont été moins stimulés chez les animaux en situation adaptée de restriction (R) relativement aux animaux nourris *ad libitum* (T). La supplémentation en souche SGCK a permis à ces deux protéines de présenter un niveau de phosphorylation proche de celui des animaux témoins (p-S6/S6) ou intermédiaires entre T et R (p-eIF2a/eIF2a).

Les résultats présentés dans les figures 5 et 6 montrent une amélioration de l'insulino-sensibilité musculaire chez les animaux supplémentés en probiotique SGCK. Par ailleurs, ces médiateurs sont impliqués dans la stimulation de la synthèse protéique liée à l'ingestion du repas. Ce mécanisme contribue à la préservation de la masse musculaire observée chez les rats supplémentés avec la souche SGCK.

### Référence à du matériel biologique déposé

Souche bactérienne de *L. casei* SGC-K déposée le 8 mars 2021 auprès de la Collection Nationale de Cultures de Microorganismes de l'Institut Pasteur, 25 rue du Docteur Roux - 75724 Paris Cedex 15 (France), sous le n° CNCM I-5663.

## Revendications

1. Composition comprenant des bactéries de la souche *L. casei* SGC-K déposée à la Collection Nationale de Cultures de Microorganismes de l'Institut Pasteur le 8 mars 2021 sous le n° CNCM I-5663.

2. Composition selon la revendication 1, dans laquelle les bactéries de la souche *L. casei* SGC-K sont combinées avec les bactéries d'une ou plusieurs autres souches bactériennes probiotiques.

3. Composition selon la revendication 2, les autres souches bactériennes probiotiques étant choisies parmi les genres *Bifidobacterium, Lactobacillus, Lactococcus, Enterococcus,* Streptococcus, *Kluyveromyces, Saccharomyces, Faecalibacterium, Blautia, Christensenella, Bacteroides, Eubacterium, Roseburia, Coprococcus* et leurs combinaisons.

4. Composition selon l'une des revendications 2 et 3, les autres souches bactériennes probiotiques étant choisies parmi les espèces *Bifidobacterium longum, Bifidobacterium lactis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus salivarius, Lactococcus lactis, Enterococcus faecium*, *Enterococcus faecalis*, *Saccharomyces cerevisiae, Saccharomyces boulardii, Faecalibacterium prausnitzii* ou leurs mélanges, de préférence choisis dans le groupe constitué par *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM I- 3446), *Bifidobacterium breve* souche A, *Lactobacillus paracasei* NCC2461 (CNCM 1-2116), *Lactobacillus johnsonii* NCC533 (CNCM 1-1225), *Lactobacillus rhamnosus* GG (ATCC53103), *Lactobacillus rhamnosus* NCC4007 (CGMCC 1. 3724), *Enterococcus faecium* SF 68 (NCC2768 ; NCIMB10415), et leurs combinaisons.

5. Composition selon l'une des revendications 1 à 4, comprenant de plus un ou plusieurs prébiotiques.

6. Composition selon la revendication 5, dans laquelle les probiotiques sont choisis parmi les oligosaccharides contenant du fructose, du galactose, du mannose ; les fibres alimentaires, en particulier les fibres fermentescibles, les fibres de soja ; l'inuline ; et leurs combinaisons.

7. Composition selon l'une des revendications 5 et 6, dans laquelle les prébiotiques sont choisis parmi les fructo-oligosaccharides (FOS), les galactooligosaccharides (GOS), les isomalto-oligosaccharides (IMO), les xylo-oligosaccharides (XOS), les arabino-xylo-oligosaccharides (AXOS), les mannan-oligosaccharides (MOS), les oligosaccharides de soja, glycosylsucrose (GS), lactosucrose (LS), lactulose (LA), palatinose-oligosaccharides (PAO), malto-oligosaccharides, les amidons résistants, les gommes et/ou hydrolysats de celles-ci, pectines et/ou hydrolysats de celles-ci, ou des combinaisons de ceux-ci.

8. Composition selon l'une des revendications 1 à 7, comprenant de plus une ou plusieurs vitamines.

9. Composition selon la revendication 8, dans laquelle les vitamines sont choisies parmi l'acide folique, la vitamine B12, la vitamine B6, vitamine A, la vitamine D, la vitamine E et la vitamine K.

10. Utilisation non-thérapeutique d'une composition selon l'une des revendications 1 à 9, pour maintenir ou accroître la masse et/ou la fonction musculaire chez un sujet en ayant besoin, en particulier chez un sujet âgé, et chez un sujet pratiquant un exercice physique intense.

11. Composition selon l'une des revendications 1 à 9, pour son utilisation en tant que médicament.

12. Composition selon l'une des revendications 1 à 9, pour son utilisation pour le traitement de la perte de masse et/ou de fonction musculaire chez un sujet en ayant besoin, en particulier chez un sujet choisi parmi les sujets en surpoids et les sujets obèses soumis à un régime alimentaire, les sujets cancéreux affectés d'une sarcopénie, les sujets diabétiques soumis à un régime alimentaire, les sujets dénutri, en particulier les sujéts âgés dénutris.

13. Composition selon l'une des revendications 1 à 9, pour son utilisation pour réduire l'insulino-résistance chez un sujet en ayant besoin, en particulier chez un sujet diabétique, et plus particulièrement un sujet affecté d'un diabète de type 2.

14. Souche *L. casei* SGC-K déposée à la Collection Nationale de Cultures de Microorganismes de l'Institut Pasteur le 8 mars 2021 sous le n° CNCM I-5663.

## Patentansprüche

1. Zusammensetzung, umfassend Bakterien des Stammes L. casei SGC-K, der am 8. März 2021 bei der Collection Nationale de Cultures de Microorganismes des Pasteur-Instituts unter der Nr. CNCM I-5663 hinterlegt wurde.

2. Zusammensetzung nach Anspruch 1, wobei die Bakterien des Stamms L. casei SGC-K mit Bakterien eines oder mehrerer anderer probiotischer Bakterienstämme kombiniert sind.

3. Zusammensetzung nach Anspruch 2, wobei die anderen probiotischen Bakterienstämme ausgewählt sind aus den Gattungen *Bifidobacterium, Lactobacillus, Lactococcus, Enterococcus, Streptococcus, Kluyveromyces, Saccharomyces, Faecalibacterium, Blautia, Christensenella, Bacteroides, Eubacterium, Roseburia, Coprococcus* und Kombinationen davon.

4. Zusammensetzung nach einem der Ansprüche 2 und 3, wobei die anderen probiotischen Bakterienstämme ausgewählt sind aus den Spezies *Bifidobacterium longum, Bifidobacterium lactis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus salivarius, Lactococcus lactis, Enterococcus faecium, Enterococcus faecalis, Saccharomyces cerevisiae, Saccharomyces boulardii, Faecalibacterium prausnitzii* oder Mischungen davon, vorzugsweise ausgewählt aus der Gruppe bestehend aus *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM 1-3446), *Bifidobacterium breve* Stamm A, *Lactobacillus paracasei* NCC2461 (CNCM 1-2116), *Lactobacillus johnsonii* NCC533 (CNCM 1-1225), *Lactobacillus rhamnosus* GG (ATCC53103), *Lactobacillus rhamnosus* NCC4007 (CGMCC 1. 3724), *Enterococcus faecium* SF 68 (NCC2768 ; NCIMB10415), und Kombinationen davon.

5. Zusammensetzung nach einem der Ansprüche 1 bis 4, ferner umfassend ein oder mehrere Präbiotika.

6. Zusammensetzung nach Anspruch 5, wobei die Probiotika ausgewählt sind aus Oligosacchariden, umfassend Fructose, Galaktose und Mannose; Ballaststoffen, insbesondere fermentierbaren Ballaststoffen, Sojabohnenfasern; Inulin; und Kombinationen davon.

7. Zusammensetzung nach einem der Ansprüche 5 und 6, wobei die Präbiotika ausgewählt sind aus Fructooligosacchariden (FOS), Galactooligosacchariden (GOS), Isomaltooligosacchariden (IMO), Xylooligosacchariden (XOS), Arabinoxylooligosacchariden (AXOS), Mannanoligosacchariden (MOS), Sojaoligosacchariden, Glykosylsaccharose (GS), Lactosaccharose (LS), Lactulose (LA), Palatinose-Oligosacchariden (PAO), Malto-Oligosacchariden, resistenten Stärken, Gummis und/oder Hydrolysaten davon, Pektinen und/oder Hydrolysaten davon oder Kombinationen davon.

8. Zusammensetzung nach einem der Ansprüche 1 bis 7, ferner umfassend ein oder mehrere Vitamine.

9. Zusammensetzung nach Anspruch 8, wobei die Vitamine ausgewählt sind aus Folsäure, Vitamin B12, Vitamin B6, Vitamin A, Vitamin D, Vitamin E und Vitamin K.

10. Nichttherapeutische Verwendung einer Zusammensetzung nach einem der Ansprüche 1 bis 9 zur Erhaltung oder Erhöhung der Muskelmasse und/oder Muskelfunktion bei einem diesbezüglich bedürftigen Individuum, insbesondere bei einem älteren Individuum, und bei einem Individuum, das intensive körperliche Betätigung ausübt.

11. Zusammensetzung nach einem der Ansprüche 1 bis 9 zur Verwendung davon als Medikament.

12. Zusammensetzung nach einem der Ansprüche 1 bis 9 für die Verwendung davon bei der Behandlung des Verlustes von Muskelmasse und/oder Muskelfunktion bei einem diesbezüglich bedürftigen Individuum, insbesondere bei einem Individuum ausgewählt aus übergewichtigen Individuen und adipösen Individuen, die einer Diät unterzogen werden, Krebspatienten, die von Sarkopenie betroffen sind, Diabetikern unter Diät, unterernährten Individuen, insbesondere unterernährten älteren Individuen.

13. Zusammensetzung nach einem der Ansprüche 1 bis 9, für die Verwendung davon zur Verringerung der Insulinresistenz bei einem diesbezüglich bedürftigen Individuum, insbesondere bei einem Diabetiker, und im Genaueren bei einem von Typ-2-Diabetes betroffenen Individuum.

14. Stamm L. casei SGC-K, der am 8. März 2021 bei der Collection Nationale de Cultures de Microorganismes des Pasteur-Instituts unter der Nr. CNCM 1-5663 hinterlegt wurde.

## Claims

1. Composition comprising bacteria of the strain L. *casei* SGC-K deposited with the Collection Nationale de Cultures de Microorganismes [French National Collection of Cultures of Microorganisms] of the Institut Pasteur on 8 March 2021 under No. CNCM I-5663.

2. Composition according to Claim 1, wherein the bacteria of the strain *L. casei* SGC-K are combined with the bacteria of one or more other probiotic bacterial strains.

3. Composition according to Claim 2, the other probiotic bacterial strains being chosen from the genera *Bifidobacterium, Lactobacillus, Lactococcus, Enterococcus, Streptococcus, Kluyveromyces, Saccharomyces, Faecalibacterium, Blautia, Christensenella, Bacteroides, Eubacterium, Roseburia, Coprococcus,* and combinations thereof.

4. Composition according to either of Claims 2 and 3, the other probiotic bacterial strains being chosen from the species *Bifidobacterium longum, Bifidobacterium lactis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus salivarius, Lactococcus lactis, Enterococcus faecium, Enterococcus faecalis, Saccharomyces cerevisiae, Saccharomyces boulardii, Faecalibacterium prausnitzii,* or mixtures thereof, preferably chosen from the group consisting of *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM 1-3446), *Bifidobacterium breve* strain A, *Lactobacillus paracasei* NCC2461 (CNCM 1-2116), *Lactobacillus johnsonii* NCC533 (CNCM 1-1225), *Lactobacillus rhamnosus* GG (ATCC53103), *Lactobacillus rhamnosus* NCC4007 (CGMCC 1. 3724), *Enterococcus faecium* SF 68 (NCC2768; NCIMB10415), and combinations thereof.

5. Composition according to one of Claims 1 to 4, additionally comprising one or more prebiotics.

6. Composition according to Claim 5, wherein the probiotics are chosen from oligosaccharides containing fructose, galactose, mannose; dietary fibres, in particular fermentable fibres, soy fibres; inulin; and combinations thereof.

7. Composition according to either of Claims 5 and 6, wherein the prebiotics are chosen from fructooligosaccharides (FOSs), galactooligosaccharides (GOSs), isomaltooligosaccharides (IMOs), xylooligosaccharides (XOSs), arabinoxylooligosaccharides (AXOSs), mannan oligosaccharides (MOSs), soybean oligosaccharides, glycosylsucrose (GS), lactosucrose (LS), lactulose (LA), palatinose oligosaccharides (PAO), maltooligosaccharides, resistant starches, gums and/or hydrolysates thereof, pectins and/or hydrolysates thereof, or combinations of these.

8. Composition according to one of Claims 1 to 7, additionally comprising one or more vitamins.

9. Composition according to Claim 8, wherein the vitamins are chosen from folic acid, vitamin B12, vitamin B6, vitamin A, vitamin D, vitamin E and vitamin K.

10. Non-therapeutic use of a composition according to one of Claims 1 to 9, for maintaining or increasing muscle mass and/or function in a subject in need thereof, in particular in an elderly subject, and in a subject doing intense physical exercise.

11. Composition according to one of Claims 1 to 9, for use thereof as a medicament.

12. Composition according to one of Claims 1 to 9, for the use thereof for the treatment of the loss of muscle mass and/or function in a subject in need thereof, in particular in a subject chosen from overweight subjects and obese subjects subjected to a diet, cancer subjects afflicted with sarcopenia, diabetic subjects subjected to a diet, malnourished subjects, in particular malnourished elderly subjects.

13. Composition according to one of Claims 1 to 9, for the use thereof for reducing insulin resistance in a subject in need thereof, in particular in a diabetic subject, and more particularly a subject afflicted with type 2 diabetes.

14. *L. casei* SGC-K strain deposited with the Collection Nationale de Cultures de Microorganismes [French National Collection of Cultures of Microorganisms] of the Institut Pasteur on 8 March 2021 under No. CNCM I-5663.
